# EUROPEAN PATENT APPLICATION

(11) **EP 4 687 152 A1**
(43) Date of publication of application: **04.02.2026**
(21) Application number: 24306303.9
(22) Date of filing: 01.08.2024
(51) Int. Cl.: G16H 20/70, G16H 40/63, G16H 50/20, G06F 40/20, G10L 25/66, G16H 30/40, G06F 40/35

(54) **DEVICE FOR COLLECTING A DATA FROM A PERSON AT RISK OF A PSYCHIATRIC DISORDER**

(71) Applicant: Emobot, 75001 Paris (FR)
(72) Inventor: PETELOT, Tanel, 75010 Paris (FR)
(74) Representative: Icosa

(57) **Abstract**

The present invention relates to a device for collecting a data from a person (P) at risk of a psychiatric disorder for a diagnostic, health monitoring and/or a screening purpose(s), said device comprising: at least one input configured to receive : at least one signal (S(tₑ)) representative of the emotional status of said person (P), said signal (S(tₑ)) being at least a voice signal of said person (P) or an image comprising at least one portion of a face of said person (P); data from a previously created knowledge database, said previously created knowledge database comprising at least one information related to said person (P) and a plurality of conversation topics, each conversation topic being associated with at least one emotion; at least one processor configured to: extract from said at least one signal (S(tₑ)) representative of the emotional status at least one emotional signature (S_{E}(tₑ)); compare said extracted at least one emotional signature (S_{E}(tₑ)) to a previous emotional signature (S_{E}(tₑ-Δt)) of said person (P) extracted at a previous time (tₑ-Δt), said previous emotional signature (S_{E}(tₑ-Δt)) being comprised in said previously created knowledge database; based on said comparison, obtain a degree of similarity of emotional signatures; based on said degree of similarity, select at least one conversation topic among said plurality of conversation topics; generate and transmit an engaging message to said person (P) based on said at least one selected conversation topic; collect, from said person (P), at least one current signal (S_{C}(t_{c})) representative of the emotional status generated after reception of said engaging message.

## Description

### FIELD OF INVENTION

The present invention relates to mental health technology and person care. In particular, the present invention relates to a device and associated method for collecting a data from a person at risk of a psychiatric disorder for a diagnostic, health monitoring and/or a screening purpose(s).

### BACKGROUND OF INVENTION

Various technologies exist today to manage and assess mental health and emotional well-being. These include empathetic chatbots (i.e. conversational agents), emotion detection systems, and personalized knowledge bases.

Chatbots are widely used for various purposes, including customer service, personal assistance, and mental health support. Some chatbots are designed to detect and mimic users' emotions in real-time to provide therapeutic support, aiming to improve users' emotional states through empathetic interactions.

Emotion detection systems are advanced systems that analyze emotions through multimodal inputs, including facial expressions, gestures, voice prosody, and textual content. These systems aim to provide real-time emotional feedback during interactions.

Personalized knowledge bases may be for example designed to build user profiles based on behavior, preferences, and interactions. Such profiles are used primarily in marketing to tailor user experiences.

Despite the advancements, existing technologies face several limitations. First, most empathetic chatbots operate on a real-time basis, reacting to users' emotions as they occur. This approach can be limited in its ability to provide a deeper, sustained emotional understanding and engagement over time. Second, existing personalized knowledge bases lack of personalized emotional context and consequently do not typically integrate an emotional dimension into the stored information. This results in a static user profile that may not fully capture the user's emotional responses to different topics or interactions. Third, many chatbots lack the capability to initiate interactions proactively based on historical emotional data, therefore function in a reactive rather than proactive engagement manner. They often wait for user input to respond, rather than actively engaging the user at opportune moments based on their emotional history. Fourth, existing systems do not effectively combine multimodal emotional data with contextual information to build a comprehensive emotional profile. This leads to fragmented emotional and contextual data that may not provide a holistic view of the user's emotional state and needs. And finally, while some systems can detect emotions through various modes (e.g., facial expressions, voice), few can synthesize and express emotions multimodally, limiting the richness of the interaction and the perceived empathy of the system.

The present invention addresses these limitations aiming to enhance the efficacy of mental health support technologies.

### SUMMARY

This invention thus relates to a device for collecting data from a person at risk of a psychiatric disorder for a diagnostic, health monitoring and/or a screening purpose(s), said device comprising:
- at least one input configured to receive:
   - at least one signal representative of an emotional status of the person, the signal being at least a voice signal of the person or an image comprising at least one portion of a face of the person;
   - data from a previously created knowledge database, the previously created knowledge database comprising at least one information related to the person and a plurality of conversation topics, each conversation topic being associated with at least one emotion;
- at least one processor configured to:
   i. extract from the at least one signal representative of the emotional status at least one emotional signature;
   ii. compare the extracted at least one emotional signature to a previous emotional signature of the person extracted at a previous time, the previous emotional signature being comprised in the previously created knowledge database;
   iii. based on said comparison, obtain a degree of similarity of emotional signatures;
   iv. based on the degree of similarity, select at least one conversation topic among the plurality of conversation topics;
   v. generate and transmit an engaging message to the person based on the at least one selected conversation topic;
   vi. collect, from the person, at least one current signal representative of the emotional status generated after reception of the engaging message.

According to other advantageous aspects of the invention, the device for collecting a data from the person comprises one or more of the features described in the following embodiments, taken alone or in any possible combination.

According to one or several embodiments, the at least processor is further configured to, after collecting the at least one current signal:
vii. extract from the at least one current signal at least one current emotional signature;
viii. update the previously created knowledge database using the at least one selected conversation topic and the at least one current emotional signature;
ix. repeat steps ii. to viii. on the at least one current signal, said at least one current signal comprising at least one among: a voice signal of the person or an image comprising at least one portion of a face of the person;
wherein said at least one output is further configured to provide the updated knowledge database.

Advantageously, the device for collecting data from a person allows to build a personalized, emotionally tagged knowledge database. Consequently, the created knowledge database enables proactive, empathetic interactions that consider the person's emotional history and context, providing more meaningful and supportive engagement during an engaged conversation based on a selected conversation topic. The device's proactivity is ensured by a continuous enrichment of the knowledge database through conversations with the person engaged at predefined, periodic or random (i.e. asynchronously) times. Indeed, providing supportive engagement during a conversation may encourage the person to communicate more with the device, eventually steering the person towards a conversation topic that simply promotes their well-being.

Advantageously, the knowledge database is personalized and signed by emotion. It compiles information extracted by the device for collecting data from a person during previous conversations between the device and the person. In other words, the emotional status of the person is analyzed to enrich the knowledge database with an emotional tag that characterizes the emotional status of the person when discussing a particular topic. This approach not only enhances the relevance and personalized nature of interactions but also enables the device to adapt conversation topics based on an emotional history of the person, fostering more empathetic and effective communication.

According to one or several embodiments, the selected conversation topic is compared to a conversation topic comprised in the previously created knowledge database to evaluate an emotional status evolution of the person.

According to one or several embodiments, the knowledge database is updated by adding a couple of information comprising the extracted at least one emotional signature and the selected at least one conversation topic to the knowledge database.

According to one or several embodiments, the extracted emotional signature is representative of: at least one emotion or a combination of at least two distinct emotions.

According to one or several embodiments, the knowledge database is structured according to a predefined ontology taking into account at least one emotional signature and at least one conversation topic.

According to one or several embodiments, the knowledge database further comprises textual, multimedia and/or contextual information associated with emotions enabling a representation of the person.

According to one or several embodiments, selecting the at least one conversation topic is based on a semantic analysis of the information comprised in the knowledge database taking into account the emotional signatures.

According to one or several embodiments, the plurality of conversation topics is updated from at least one external data source.

According to one or several embodiments, the at least one input is further configured to receive at least one initial signal representative of the emotional status of the person, the initial signal being at least a voice signal of the person or an image comprising at least a portion of the face of the person; and the at least one processor is further configured to:
- extract from the at least one initial signal at least one initial emotional signature;
- add the initial emotional signature to the previously created knowledge database.

According to one or several embodiments, the at least one processor is further configured to:
- periodically select a predefined question from a list of predefined questions;
- generate and transmit an engaging message to the person based on the predefined question;
- collect, from the person, at least one current signal representative of the emotional status generated after reception of the engaging message to the person based on the predefined question, and extract from the at least one current signal at least one current emotional signature;
- update the previously created knowledge database using the predefined question and the at least one current emotional signature.

According to one or several embodiments, the at least one signal representative of the emotional status of the person further comprises a motion signal obtained using an inertial unit worn by the person.

According to one or several embodiments, the device is further configured to receive as input at least one result from at least one questionnaire for evaluating a psychiatric status of the person, and wherein the at least one result is used to update the knowledge database.

According to one or several embodiments, the at least one information related to the person is at least one of the following: age, sex, weight, height, hobby, preference, preferred sport, health data.

The present disclosure further relates to a computer-implemented method for collecting a data from a person at risk of a psychiatric disorder for a diagnostic, health monitoring and/or a screening purpose(s), the method comprising:
a) receiving:
   - at least one signal representative of an emotional status of the person, the signal being at least a voice signal of the person or an image comprising at least one portion of a face of the person;
   - data from a previously created knowledge database, the previously created knowledge database comprising at least one information related to the person and a plurality of conversation topics, each conversation topic being associated with at least one emotion;
b) extracting from the at least one signal representative of the emotional status at least one emotional signature;
c) comparing the extracted at least one emotional signature to a previous emotional signature of the person extracted at a previous time, the previous emotional signature being comprised in the previously created knowledge database;
d) based on the comparison, obtaining a degree of similarity of emotional signatures;
e) based on the degree of similarity, selecting at least one conversation topic among the plurality of conversation topics;
f) generating and transmitting an engaging message to the person based on the at least one selected conversation topic;
g) collecting, from the person, at least one current signal representative of the emotional status generated after reception of the engaging message.

According to one or several embodiments, the computer-implemented method further comprises:
h) extracting from the at least one current signal at least one current emotional signature;
i) updating the previously created knowledge database using the at least one selected conversation topic and the at least one current emotional signature;
j) repeating steps c) to i) on said at least one current signal while the at least one current signal comprises at least one among: a voice signal of the person (P) or an image comprising at least one portion of a face of the person;
k) providing the updated knowledge database.

In addition, the disclosure relates to a computer program comprising software code adapted to perform a method for collecting a data from a person at risk of a psychiatric disorder compliant with any of the above execution modes when the program is executed by a processor.

The present disclosure further pertains to a non-transitory program storage device, readable by a computer, tangibly embodying a program of instructions executable by the computer to perform a method for collecting a data from a person at risk of a psychiatric disorder, compliant with the present disclosure.

Such a non-transitory program storage device can be, without limitation, an electronic, magnetic, optical, electromagnetic, infrared, or semiconductor device, or any suitable combination of the foregoing. It is to be appreciated that the following, while providing more specific examples, is merely an illustrative and not exhaustive listing as readily appreciated by one of ordinary skill in the art: a portable computer diskette, a hard disk, a ROM, an EPROM (Erasable Programmable ROM) or a Flash memory, a portable CD-ROM (Compact-Disc ROM).

### DEFINITIONS

In the present invention, the following terms have the following meanings:

The terms **"adapted"** and **"configured"** are used in the present disclosure as broadly encompassing initial configuration, later adaptation or complementation of the present device, or any combination thereof alike, whether effected through material or software means (including firmware).

The term **"processor"** should not be construed to be restricted to hardware capable of executing software, and refers in a general way to a processing device, which can for example include a computer, a microprocessor, an integrated circuit, or a programmable logic device (PLD). The processor may also encompass one or more Graphics Processing Units (GPU), whether exploited for computer graphics and image processing or other functions. Additionally, the instructions and/or data enabling to perform associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.

**"Machine learning (ML)"** designates in a traditional way computer algorithms improving automatically through experience, on the ground of training data enabling to adjust parameters of computer models through gap reductions between expected outputs extracted from the training data and evaluated outputs computed by the computer models.

A **"neural network (NN)"** designates a category of ML comprising nodes (called **"neurons"**), and connections between neurons modeled by **"weights".** For each neuron, an output is given in function of an input or a set of inputs by an **"activation function".** Neurons are generally organized into multiple **"layers",** so that neurons of one layer connect only to neurons of the immediately preceding and immediately following layers.

An **"ontology"** is a set of concepts organized in a hierarchical manner and pertains to a specific domain. For example, the concept "family" can consist of the sub-concepts "man," "woman," "child," and "adult." There are relationships between these concepts, such as the "part of" relationship. For instance, a "woman" is part of the "family." Each concept is associated with a set of properties: name, age, main activity. For example, the name (a property of the concept woman) of the woman is X (the value of the property).

The above ML definitions are compliant with their usual meaning, and can be completed with numerous associated features and properties, and definitions of related numerical objects, well known to a person skilled in the ML field. Additional terms will be defined, specified or commented wherever useful throughout the following description.

### BRIEF DESCRIPTION OF THE DRAWINGS

The present disclosure will be better understood, and other specific features and advantages will emerge upon reading the following description of particular and non-restrictive illustrative embodiments, the description making reference to the annexed drawings wherein:
**Figure 1** is a block diagram representing schematically a particular mode of a device for collecting a data from a person at risk of a psychiatric disorder, compliant with the present disclosure;
**Figure 2** is a flow chart showing successive steps of a computer-implemented method executed with the device for collecting a data from a person at risk of a psychiatric disorder of figure 1;
**Figure 3** is a block diagram representing schematically a particular mode of the device of figure 1, compliant with the present disclosure;
**Figure 4** shows an apparatus integrating the functions of the device for collecting a data from a person at risk of a psychiatric disorder of figure 1.

On the figures, the drawings are not to scale, and identical or similar elements are designated by the same references.

### ILLUSTRATIVE EMBODIMENTS

The present description illustrates the principles of the present disclosure. It will thus be appreciated that those skilled in the art will be able to devise various arrangements that, although not explicitly described or shown herein, embody the principles of the disclosure and are included within its scope.

All examples and conditional language recited herein are intended for educational purposes to aid the reader in understanding the principles of the disclosure and the concepts contributed by the inventor to furthering the art, and are to be construed as being without limitation to such specifically recited examples and conditions.

Moreover, all statements herein reciting principles, aspects, and embodiments of the disclosure, as well as specific examples thereof, are intended to encompass both structural and functional equivalents thereof. Additionally, it is intended that such equivalents include both currently known equivalents as well as equivalents developed in the future, i.e., any elements developed that perform the same function, regardless of structure.

Thus, for example, it will be appreciated by those skilled in the art that the block diagrams presented herein may represent conceptual views of illustrative circuitry embodying the principles of the disclosure. Similarly, it will be appreciated that any flow charts, flow diagrams, and the like represent various processes which may be substantially represented in computer readable media and so executed by a computer or processor, whether or not such computer or processor is explicitly shown.

The functions of the various elements shown in the figures may be provided through the use of dedicated hardware as well as hardware capable of executing software in association with appropriate software. When provided by a processor, the functions may be provided by a single dedicated processor, a single shared processor, or a plurality of individual processors, some of which may be shared.

It should be understood that the elements shown in the figures may be implemented in various forms of hardware, software or combinations thereof. Preferably, these elements are implemented in a combination of hardware and software on one or more appropriately programmed general-purpose devices, which may include a processor, memory and input/output interfaces.

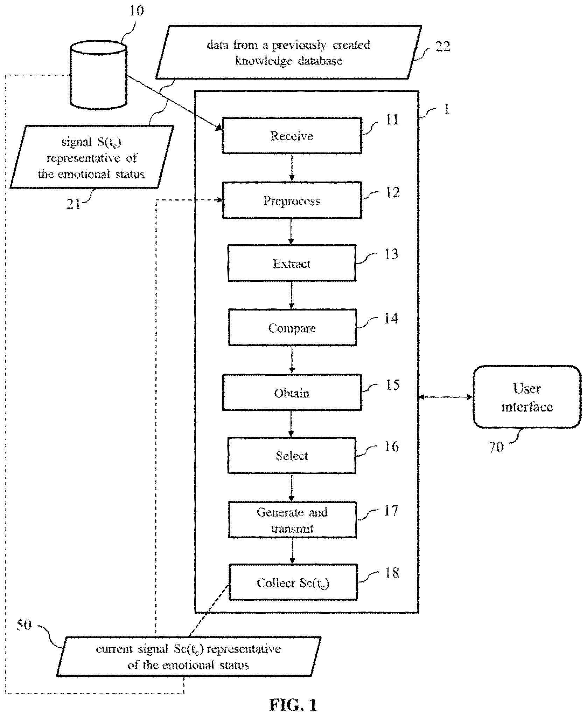

The present disclosure will be described in reference to a particular functional embodiment of a device 1 for collecting a data from a person P at risk of a psychiatric disorder for a diagnostic, health monitoring and/or a screening purpose(s), as illustrated on **Figure 1****.**

The device 1 is adapted to collect, from the person P, at least one current signal S_{C}(t_{c}) 50 representative of an emotional status generated after reception of an engaging message.

The device 1 comprises at least one input configured to receive 11:
- at least one signal S(tₑ) 21 representative of the emotional status of the person P, the signal S(tₑ) 21 being at least a voice signal of the person P or an image comprising at least one portion of a face of the person P;
- data 22 from a previously created knowledge database, the previously created knowledge database comprising at least one information related to the person P and a plurality of conversation topics, each conversation topic being associated with at least one emotion.

Though the presently described device 1 is versatile and provided with several functions that can be carried out alternatively or in any cumulative way, other implementations within the scope of the present disclosure include devices having only parts of the present functionalities.

The device 1 is advantageously an apparatus, or a physical part of an apparatus, designed, configured and/or adapted for performing the mentioned functions and produce the mentioned effects or results. In alternative implementations, the device 1 is embodied as a set of apparatus or physical parts of apparatus, whether grouped in a same machine or in different, possibly remote, machines. The device 1 may e.g. have functions distributed over a cloud infrastructure and be available to users as a cloud-based service, or have remote functions accessible through an API.

In what follows, the modules are to be understood as functional entities rather than material, physically distinct, components. They can consequently be embodied either as grouped together in a same tangible and concrete component, or distributed into several such components. Also, each of those modules is possibly itself shared between at least two physical components. In addition, the modules are implemented in hardware, software, firmware, or any mixed form thereof as well. They are preferably embodied within at least one processor of the device 1.

The device 1 comprises a module 11 for receiving at least one signal S(tₑ) 21 representative of the emotional status of the person P and the data 22 from a previously created knowledge database stored in one or more local or remote database(s) 10. The database(s) 10 may be used to store the collected current signal S_{C}(t_{c}) 50. Note that t_{c} corresponding to a time following tₑ. The latter can take the form of storage resources available from any kind of appropriate storage means, which can be notably a RAM or an EEPROM (Electrically-Erasable Programmable Read-Only Memory) such as a Flash memory, possibly within an SSD (Solid-State Disk). In some embodiments, the at least one signal S(tₑ) 21 representative of the emotional status of the person P and the data 22 from a previously created knowledge database may be received from an external or peripheral device.

The signal S(tₑ) 21 representative of the emotional status of the person P may be at least one among: a voice signal of the person P, an image comprising at least one portion of a face of the person P, a text input from a chat associated to the person P, a physiological signal (e.g. heart rate, blood pressure), a gesture of the person P. The signal S(tₑ) representative of the emotional status may be one or several of the previously mentioned options, or even a possible combination of them, underlining the multimodality capacities of the device 1. Each signal S(tₑ) 21 representative of the emotional status may be obtained or collected from an adequate sensor. For example, the voice signal of the person P may be collected from a phone or tablet microphone. An image comprising at least one portion of the face of the person P may be acquired using a phone camera or a peripheral camera connected to the device 1. A text input from a chat associated to the person P may be obtained from a smartphone or a smartwatch. A gesture of the person P may be obtained from a motion sensor (i.e., inertial unit), a wearable device or an image(s) analysis acquired with a camera.

The previously created knowledge database, at the initialization moment (i.e., before first use from the user/person P) may be a prefilled or an empty database that may be configured to be updated continuously, periodically or at random times. The knowledge database may be configured to comprise at least one information related to the person P and a plurality of conversation topics, each conversation topic being associated with at least one emotion. The information related to the person P may be a hobby of the person P, a preference of the person P, a preferred sport of the person P, the age of the person P, the sex of the person P, the weight/height of the person P, a health data of the person P, or any other information relative to the person's P. Here is a non-exhaustive list of examples of conversation topics and associated emotion(s):
- a conversation topic may be talking about a recent achievement; associated emotion(s) may be: pride, happiness;
- a conversation topic may be discussing a personal failure; associated emotion(s) may be: disappointment, sadness;
- a conversation topic may be sharing a joke; associated emotion(s) may be: joy, amusement;
- a conversation topic may be talking about a recent achievement; associated emotion(s) may be: pride, happiness;
- a conversation topic may be talking about a stressful event; associated emotion(s) may be: stress, frustration;
- a conversation topic may be discussing actual events; associated emotion(s) may be: concern, interest;
- a conversation topic may be planning a vacation; associated emotion(s) may be: excitement, anticipation;
- a conversation topic may be talking about health issues; associated emotion(s) may be: worry, anxiety.

In one embodiment, the device 1 may be first used by the person P in case no data collection was previously done from the person P. In this case, the knowledge database may be newly created for the person P. This knowledge database may be filled progressively during time with each data collection from the person P.

The device 1 may also be configured to initiate interactions proactively based on historical emotional data (i.e. data comprised in the knowledge database) at any time of the day. In this case, the device is configured to analyze the environment and cross-references this environment analysis with the knowledge database to initiate a conversation based on a selected conversation topic. The module 11 may be further configured to receive external data source. For example, an external data source may be news feeds, social media platforms, weather APIs, calendar applications, location-based services, industry reports, health and fitness trackers, government databases, entertainment databases, educational platforms. For instance, if the person P enjoys chess and the device 1 identifies from an external data source that a local organization nearby is hosting "chess evenings", the device 1 may generate and transmit an engaging message to the person P to get to know if the person P is interested in participating in such an event.

The device 1 further comprises optionally a module 12 for preprocessing the received at least one signal S(tₑ) 21 representative of the emotional status of the person P and the data 22 from the previously created knowledge database. The module 12 may notably be adapted to perform standardization, normalization, filtering and/or features extraction tasks on received at least one signal S(tₑ) 21 representative of the emotional status of the person P and/or the data 22 from a previously created knowledge database. According to various configurations, the module 12 is adapted to execute only part or all of the above functions, in any possible combination, in any manner suited to the following processing stage.

In advantageous modes, the module 12 is configured for preprocessing the received at least one signal S(tₑ) 21 representative of the emotional status of the person P and the data 22 from a previously created knowledge database so as to have the received at least one signal S(tₑ) 21 and the data 22 standardized. This may enhance the efficiency of the downstream processing by the device 1. Such a standardization may be particularly useful when exploited at least one signal S(tₑ) 21 and the data 22 originate from different sources (e.g. different formats, various protocols, multiple platforms, heterogeneous systems). Thanks to standardization, differences between sources may then be neutralized or minimized. This may make the device 1 more efficient and reliable.

The device 1 may further comprise a module 13 for extracting, from the at least one signal S(tₑ) 21 representative of the emotional status, at least one emotional signature S_{E}(tₑ).

In case the signal S(tₑ) 21 representative of the emotional status is a voice signal, features such as tone, pitch, formants and/or speech rate may be obtained. Voice signal(s) may be analyses and/or processed with formants and/or all the classic audio analysis techniques that aim to provide audio features (statistical tools, Machine Learning), including deep neural networks such as Transformers, for example. The at least one emotional signature S_{E}(tₑ) may be extracted from the obtained features. In another example, where the signal S(tₑ) 21 is an image comprising at least one portion of a face of the person P, features such as facial landmarks may be obtained. If the signal S(tₑ) 21 is a gesture of the person P, features such as speed and/or direction of the gesture may be obtained.

Based on the type and the source of the signal S(tₑ) 21, an adequate method may be applied to obtain the features.

For example, if the signal S(tₑ) 21 is an image of the face of the person P obtained from a phone camera, a neural network (e.g. a convolutional neural network (CNN), a residual neural network, a transformer, a masked transformer) may be used to obtain the facial features and extract the at least one emotional signature S_{E}(tₑ).

In another example, if the signal S(tₑ) 21 is a voice signal of the person P obtained from a microphone, methods such as Mel-Frequency Cepstral Coefficients (MFCCs) extraction or prosodic feature analysis may be used to analyze the voice signal and obtain the features. The obtained features may be then used by a machine learning technique (e.g., support vector machine, neural network) or a statistical model (e.g., gaussian mixture model) to extract the at least one emotional signature S_{E}(tₑ).

In case the signal S(tₑ) 21 is a gesture of the person P obtained from a motion sensor, techniques such as kinematic analysis or the use of Hidden Markov Models (HMMs) may be used to analyze the motion data and extract features from the gesture. The extracted features may be then used by a machine learning technique (e.g., decision trees, random forests) or a pattern recognition algorithm (e.g., Hidden Markov Models) to obtain the at least one emotional signature S_{E}(tₑ).

An emotional signature may be represented by a feature vector or array that comprises a combination of features obtained from a signal S(tₑ) representative of the emotional status.

The device 1 may further comprise a module 14 for comparing the extracted at least one emotional signature S_{E}(tₑ) to a previous emotional signature S_{E}(tₑ-Δt) of the person P extracted at a previous time tₑ-Δt. The comparison may comprise a preprocessing step to ensure both emotional signatures (e.g., S_{E}(tₑ) and S_{E}(tₑ-Δt)) are in a same format. For instance, the preprocessing step may comprise normalizing feature vectors if they are not already normalized to ensure that the comparison is scale-independent. Then, the comparison may be done using a distance metric between feature vectors corresponding to emotional signatures. The distance metric may be an Euclidian distance, a Cosine similarity, a Manhattan distance. Feature vectors may be compared over time to identify trends or patterns. This may be done using time-series analysis techniques such as moving averages or exponential smoothing.

The device 1 may further comprise a module 15 for obtaining (i.e., calculating) a degree of similarity of emotional signatures based on the comparison of emotional signatures. A threshold may be defined to detect significant changes in the emotional status of the person. For example, if the Euclidean distance exceeds a certain threshold, it may indicate a significant emotional change. The threshold may be a predefined threshold (e.g. based on literature or on experimental evidence), a fixed threshold or an adjustable threshold which value changes with time. The degree of similarity may be obtained from a difference between the threshold and the distance between feature vectors. Indeed, the degree of similarity may indicate how closely a current emotional state matches a previous emotional state, in other words the extent of resemblance between a current emotional signature and a previous one. For instance, a value of +1 may indicate a maximum of similarity between the current and the previous emotional signatures; this may indicate that the current and the previous emotional signatures are identical. In opposition, a value of -1 may indicate that the current and the previous emotional signatures are completely different (e.g. joy vs sadness, optimistic vs pessimistic).

The device 1 may further comprise a module 16 for selecting at least one conversation topic based on the degree of similarity. Let's consider an example in which are available two different emotional signatures corresponding to two opposite emotions (a negative emotion indicating an unhealthy emotional status vs a positive emotion indicating a healthy emotional status), e.g. optimistic (positive emotion) vs pessimistic (negative emotion). In this case, the degree of similarity may indicate a minimal value of resemblance between emotional signatures (e.g. -1). In case the previous emotional signature S_{E}(tₑ-Δt) corresponds to the positive emotion and the emotional signature S_{E}(tₑ) corresponds to the negative emotion, the conversation topic may be chosen to improve the well-being of the person P. For example, the device might suggest a conversation topic such as "Gratitude Practices," "Positive Memories," or "Achievements and Success Stories" to help shift the person's emotional status from pessimism towards optimism, thereby fostering a healthier emotional balance.

In another example, the conversation topic may be selected to keep the person P interested and engaged by focusing on areas of their known interests. For instance, if person P enjoys hobbies such as cooking, a conversation topic such as "New Recipes to Try" may be selected.

In some cases, a positive emotion may be a neutral state and a negative emotion may be an exaggerated joy. For instance, in the person P has bipolar disease, it is sometimes better to return to a neutral emotional level (i.e. in this example this corresponds to the positive emotion) rather than being exaggeratedly joyful (i.e. in this example this corresponds to the negative emotion), the conversation topic may be chosen to stabilize the emotional status of the person P. If the person P is feeling exaggeratedly joyful, the device might suggest a conversation topic such as "Mindfulness Techniques," "Neutral Daily Activities," or "Balanced Lifestyle Tips" to help shift the person's emotional status towards a neutral, balanced state, thereby promoting emotional stability and well-being.

The device 1 may further comprise a module 17 for generating and transmitting an engaging message to the person P based on the at least one selected conversation topic.

For example, if the selected conversation topic is "Planning a family vacation", module 17 may generate a personalized and encouraging message and transmit it to the phone of the person P. This message may read, "Hi [Name of person P], I know things might feel tough right now, but let's think about something exciting to look forward to. How about planning a family vacation? Where would you and your loved ones like to go? Maybe a beach getaway, or exploring a new city? Fun activities and memorable experiences to be shared with your family are on the way!" The message could be transmitted through the person's preferred communication channel, such as a text message, email, or app notification, ensuring it is received in a timely and accessible manner. The message may be a text or a voice message, and the module 17 may adapt the message tone and content based on the historical responses and preferences of the person P, ensuring that the communication is empathetic and engaging. A preference of the person P may be receiving a voice message if the person P is not in a condition that allows him to read a text message. In a different scenario, the device 1 may be a proactive and empathetic chatbot; the person P may be at home and an engaging voice message is generated and transmitted by the chatbot.

The device 1 may further comprise a module 18 for collecting, from the person P, at least one current signal S_{C}(t_{c}) 50 representative of the emotional status generated after reception of the engaging message, which advantageously allow to capture information on the emotional reaction of the person raised by the conversion topic. In one example, the module 18 may collect a current signal S_{C}(t_{c}) 50 such as an image captured by a camera associated to the device 1. A current emotional signature S_{CE}(t_{c}) may be extracted from the current signal S_{C}(t_{c}) 50, for example from analyzing the facial expressions of the person P after receiving the engaging message. As this current emotional signature S_{CE}(t_{c}) may provide real-time insights into how the person P felt after receiving the engaging message, the device 1 advantageously adapts its interaction strategy dynamically based on emotional cues (i.e. changes from S_{E}(tₑ-Δt), to S_{E}(tₑ), to S_{CE}(t_{c})). The current emotional signature S_{CE}(t_{c}) may be stored in the database 10.

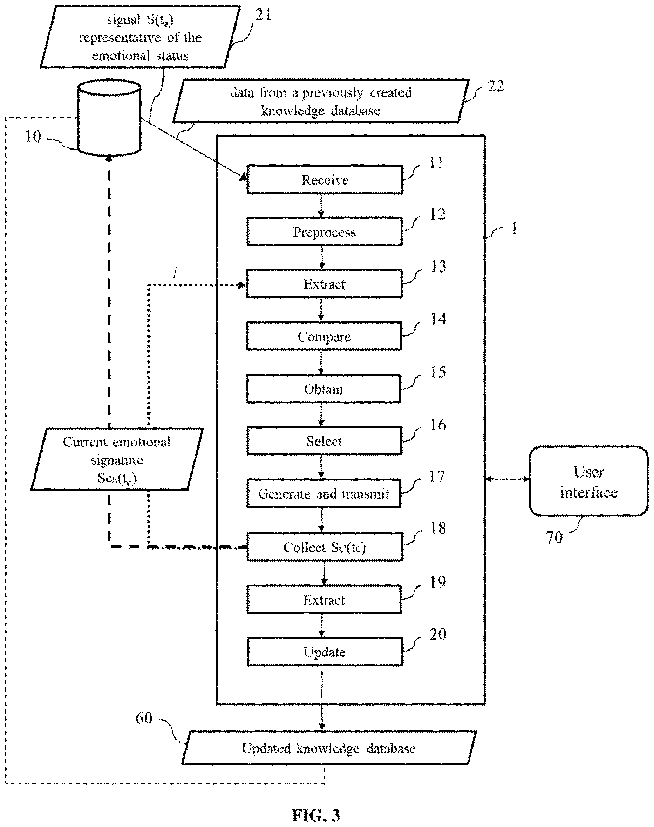

As illustrated on **Figure 3****,** the device 1 may further comprise a module 19 for extracting from the at least one current signal S_{C}(t_{c}) 50 at least one current emotional signature S_{CE}(t_{c}). Note that, everything that may apply to the signal S(t_{c}) 21 may also apply to the current signal S_{C}(t_{c}) 50. Similarly, everything that may apply to the emotional signature S_{E}(t_{c}) may also apply to the current emotional signature S_{CE}(t_{c}). In other words, the current signal S_{C}(t_{c}) 50 may be comprised in the at least one input comprised in the device 1; the processor may then extract from the current signal S_{C}(t_{c}) 50 the current emotional signature S_{CE}(t_{c}); and then, the processor may continue by applying the steps of modules 14, 15, 16 and 17 to the current signal S_{C}(t_{c}) 50 and the current emotional signature S_{CE}(t_{c}), to collect (module 18) from the person P, at least one signal S(t_{c}+Δt) representative of the emotional status generated after reception of an engaging message, t_{c}+Δt corresponding to an instant following t_{c} (i.e. actual/current time). For instance, the emotional signature S_{E}(tₑ) may be extracted before the at least one current emotional signature S_{CE}(t_{c}); similarly, the emotional signature S_{CE}(t_{c}) may be extracted before at least one further emotional signature S_{fE}(t) extracted from the at least one further signal S_{f}(t) representative of the emotional status. A previous time tₑ-Δt may be any time within the last minute/hour, the same day, the last week/week/year, any time during a specific event or context, any time since the last emotional change was detected, any time within a custom time frame previously specified, or any time during a recurring situation or routine (e.g., morning routines, work hours).

The device 1 may further comprise a module 20 for updating the previously created knowledge database using the at least one selected conversation topic and the at least one current emotional signature S_{CE}(t_{c}) 50. This update may involve combining the selected conversation topic with the current emotional signature S_{CE}(t_{c}) and relevant contextual information (e.g., time, date, activity). It is referred to this combination as a "new data entry" for the knowledge database. The new data entry may then be added to an appropriate table within the knowledge database, ensuring it adheres to a predefined ontology by maintaining standardized relationships and hierarchies, facilitating accurate data retrieval and integration using machine learning models for instance. This may allow for trend analysis over time, enhancing the system's predictive accuracy. The predefined ontology may comprise The updated knowledge database may be used, by other devices, to train machine learning models, refining ability of those devices to predict the emotional impact of various conversation topics. The effectiveness of a conversation topic in improving the emotional status of the person P may be monitored, with feedback used to make further adjustments, ensuring the device 1 remains responsive and empathetic to the need of the person P.

The predefined ontology may comprise concepts and associated sub-concepts. For example, the concept "emotional status" can consist of sub-concepts like "happy," "sad," "anxious," and "calm." There might be relationships between these sub-concepts, such as a "transition to" relationship. For instance, "sad" can transition to "happy". Each sub-concept may be associated with a set of properties such as intensity and duration. The intensity may be measured on a scale from 1 to 10. The duration may be measured in units of time such as seconds, minutes, hours, or days.

Another concept may be a "conversation topic" consisting of sub-concepts such as "hobbies," "health," "work," and "family." There are relationships between concepts and sub-concepts, such as a "relevant to" relationship. For instance, "hobbies" is relevant to the "emotional status" of a person. Each sub-concept may be associated with a set of properties such as topic name and associated emotion. For example, the associated emotion (a property of the sub-concept hobbies) could be "joy" when discussing hobbies like painting (i.e. topic name in this example).

The device 1 may be configured to iteratively (after reception of a "first" signal S(tₑ), i.e. a signal S(tₑ) representative of the emotional status): collect a "new" current signal S_{C}(t_{c}) with module 18, then analyze it using modules (optionally 12), 13, 14, 15,16 and 17 (i.e., extract the current emotional signature S_{CE}(t_{c}), compare etc.) and update the knowledge database using module 20, then start again the iteration from the step of comparison, implemented by module 14, using the current emotional signature S_{CE}(t_{c}).

The device 1 may be as well configured to iteratively perform the steps implemented by modules (12 or) 13 to 18, without updating the knowledge database at all, or without updating the knowledge database at every iteration.

In one embodiment, a threshold may be defined to decide whether, after collecting the at least one current signal S_{C}(t_{c}) 50 by module 18:
- to store the collected current signal S_{C}(t_{c}) 50 in the database 10; or
- to extract a current emotional signature S_{CE}(t_{c}) (by module 19) and update the knowledge database (by module 20); or
- to discard the collected current signal S_{C}(t_{c}) 50; or
- to reiterate on the collected current signal S_{C}(t_{c}) 50 using modules (optionally 12), 13, 14, 15, 16, 17, 18.

The threshold for deciding may be chosen for example based on a quality parameter associated to the collected current signal S_{C}(t_{c}) 50. Such a quality parameter may be a signal-to-noise ratio (SNR) or a signal strength indicator (SSI) for instance.

The plurality of conversation topics may be created and/or updated from an external data source (such as the external data sources mentioned earlier). Furthermore, the plurality of conversation topics may be updated from each signal representative of the emotional status 21 collected from the person P during the utilization of the device 1. For example, if a conversation topic has been associated to an emotional signature corresponding to a negative emotion in the knowledge database, this conversation topic may not be selected if the person P is already in a negative emotional status. However, a potential conversation topic that may be chosen is one that may help shift the person's emotional status towards a more positive status. For instance, a conversation topic such as "Solitude in Life" may have been associated with "sadness and loneliness" in the knowledge database; this conversation topic may not be selected if the person P is already feeling alone. However, a potential conversation topic that may be chosen is "Planning a family vacation," which may help the person P to feel more surrounded and supported by others.

The device 1 may further be configured for providing the updated knowledge database.

In the device 1, the processor may select a predefined question from a list of predefined questions, and then generate and transmit an engaging message to the person P based on the predefined question. The predefined question may be any relevant question allowing to get to know more the person P. For example, the predefined question may be about the person's age, family composition, hobbies, dietary preferences, educational background, career aspirations, familial relationships, travel experiences, cultural interests, personal values, health habits, technological preferences, and other relevant details to better understand their preferences and interests.

Let's consider an example in which P1 is the best friend of person P and that P1 is associated with a positive emotion such as "joy". The device 1 may have the capacity to retrieve some temporal information related to a communication with the person P (e.g. time since last conversation with the person). For instance, if the time since the last conversation with the person P exceeds a certain threshold (e.g. a week), the device 1 may initiate a conversation (e.g. transmit an engaging message) with a predefined question to the person P such as "How is P1 doing?" or "Have you heard from P1 recently?".

For a more user-friendly experience, the device 1 may be represented by various forms, ranging from a text-based chatbot to a voice interface, a 2D or 3D character, or any other avatar representation.

It may be observed that the operations by the modules 11, 12, 13, 14, 15, 16, 17, 18, 19 and 20are not necessarily successive in time, and may overlap, proceed in parallel or alternate, in any appropriate manner.

The device 1 may interact with a user interface 70, via which information can be entered and retrieved by the person P. The user interface 70 includes any means appropriate for entering or retrieving data, information or instructions, notably visual, tactile and/or audio capacities that can encompass any or several of the following means as well known by a person skilled in the art: a screen, a keyboard, a trackball, a touchpad, a touchscreen, a loudspeaker, a voice recognition system.

Several tools may assist the device 1. Such tools may come from the fields of Artificial Intelligence and Machine Learning, including CNNs (Convolutional Neural Networks), DVAEs (Dynamic Variational Auto-Encoders), VAEs (Variational Auto-Encoders), dialogue generators (such as generative models like Large Language Models LLMs or Generative Pre-trained Transformers GPTs), transformers and their variants (e.g. masked transformers, vision transformers) and decision trees.

In some embodiments, the device 1 may be further configured to receive as input at least one result from a questionnaire for evaluating a psychiatric status of the person P. Examples of such questionnaires: the Beck Depression Inventory (BDI) for assessing depression levels, the Generalized Anxiety Disorder 7 (GAD-7) for measuring anxiety, the Mood Disorder Questionnaire (MDQ) for identifying symptoms of bipolar disorder, the Patient Health Questionnaire (PHQ-9) for depression screening, and the PTSD Checklist (PCL-5) for evaluating post-traumatic stress disorder. The results of such questionnaires provided as input to the device 1 enable the device 1 to tailor interactions based on the mental health status of the person P. The questions from a questionnaire may be integrated into the list of predefined questions so that, over time, all the questions from a questionnaire can be asked, and a score relative to a psychiatric disorder may be obtained.

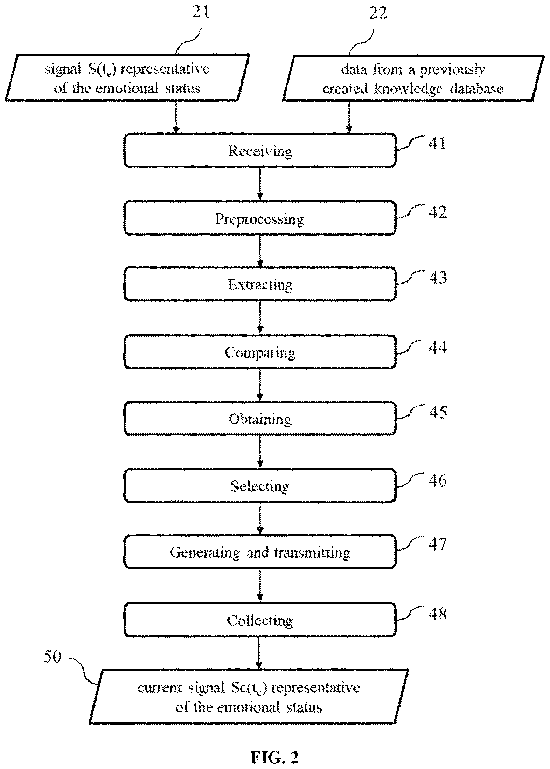

In its automatic actions, the device 1 may for example execute the following process (**Figure 2****):**
- receiving 41:
   - at least one signal S(tₑ) 21 representative of an emotional status of the person P, the signal S(tₑ) 21 being at least a voice signal of the person P or an image comprising at least one portion of a face of the person P;
   - data 22 from a previously created knowledge database, the previously created knowledge database comprising at least one information related to the person P and a plurality of conversation topics, each conversation topic being associated with at least one emotion;

- extracting 43 from the at least one signal S(tₑ) 21 representative of the emotional status at least one emotional signature S_{E}(tₑ);
- comparing 44 the extracted at least one emotional signature S_{E}(tₑ) to a previous emotional signature S_{E}(tₑ-Δt) of the person P extracted at a previous time tₑ-Δt, the previous emotional signature S_{E}(tₑ-Δt) being comprised in the previously created knowledge database;
- based on said comparison, obtaining 45 a degree of similarity of emotional signatures;
- based on the degree of similarity, selecting 46 at least one conversation topic among the plurality of conversation topics;
- generating and transmitting 47 an engaging message to the person P based on the at least one selected conversation topic;
- collecting 48, from the person P, at least one current signal SC(tc) 50 representative of the emotional status generated after reception of the engaging message.

The process (of **Figure 2**) may optionally comprise a preprocessing step 42 of the at least one signal S(tₑ) 21 and/or the data 22.

The preprocessing step 42 may be similar to a step executed by the preprocessing module 12.

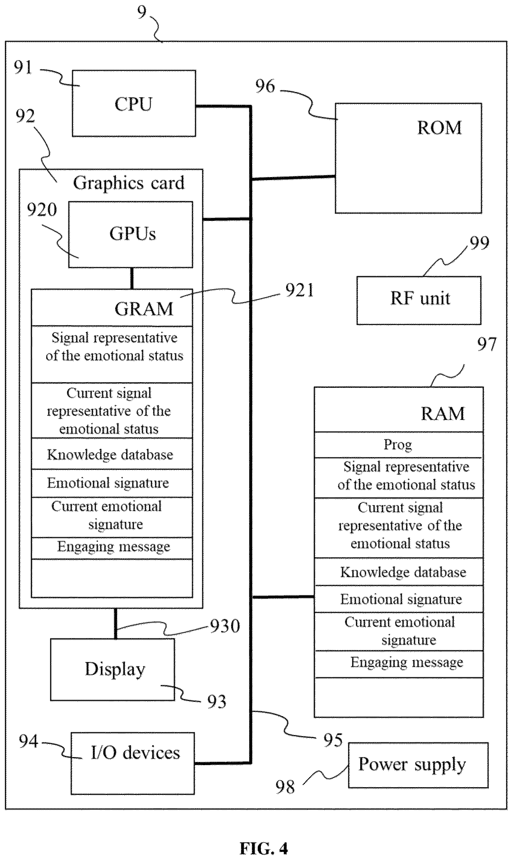

A particular apparatus 9, visible on Figure 4, is embodying the device 1 as described above. It corresponds for example to a workstation, a laptop, a tablet, a smartphone, a chatbot, or a head-mounted display (HMD).

That apparatus 9 is suited for collecting a data from a person P at risk of a psychiatric disorder for a diagnostic, health monitoring and/or a screening purpose(s). It comprises the following elements, connected to each other by a bus 95 of addresses and data that also transports a clock signal:
- a microprocessor 91 (or CPU);
- a graphics card 92 comprising several Graphical Processing Units (or GPUs) 920 and a Graphical Random Access Memory (GRAM) 921; the GPUs are quite suited to signal processing, due to their highly parallel structure;
- a non-volatile memory of ROM type 96;
- a RAM 97;
- one or several I/O (Input/Output) devices 94 such as for example a keyboard, a mouse, a trackball, a webcam; other modes for introduction of commands such as for example vocal recognition are also possible;
- a power source 98; and
- a radiofrequency unit 99.

According to a variant, the power supply 98 is external to the apparatus 9.

The apparatus 9 also comprises a display device 93 of display screen type directly connected to the graphics card 92 to display synthesized images calculated and composed in the graphics card. The use of a dedicated bus to connect the display device 93 to the graphics card 92 offers the advantage of having much greater data transmission bitrates and thus reducing the latency time for the displaying of images composed by the graphics card. According to a variant, a display device is external to apparatus 9 and is connected thereto by a cable or wirelessly for transmitting the display signals. The apparatus 9, for example through the graphics card 92, comprises an interface for transmission or connection adapted to transmit a display signal to an external display means such as for example an LCD or plasma screen or a video-projector. In this respect, the RF unit 99 can be used for wireless transmissions.

It is noted that the word "register" used hereinafter in the description of memories 97 and 921 can designate in each of the memories mentioned, a memory zone of low capacity (some binary data) as well as a memory zone of large capacity (enabling a whole program to be stored or all or part of the data representative of data calculated or to be displayed). Also, the registers represented for the RAM 97 and the GRAM 921 can be arranged and constituted in any manner, and each of them does not necessarily correspond to adjacent memory locations and can be distributed otherwise (which covers notably the situation in which one register includes several smaller registers).

When switched-on, the microprocessor 91 loads and executes the instructions of the program contained in the RAM 97.

As will be understood by a skilled person, the presence of the graphics card 92 is not mandatory, and can be replaced with entire CPU processing and/or simpler visualization implementations.

In variant modes, the apparatus 9 may include only the functionalities of the device 1. In addition, the device 1 may be implemented differently than a standalone software, and an apparatus or set of apparatus comprising only parts of the apparatus 9 may be exploited through an API call or via a cloud interface.

## Claims

1. A device (1) for collecting data from a person (P) at risk of a psychiatric disorder for a diagnostic, health monitoring and/or a screening purpose(s), said device comprising:
- at least one input configured to receive (11):
• at least one signal (S(tₑ)) (21) representative of an emotional status of said person (P), said signal (S(tₑ)) (21) being at least a voice signal of said person (P) or an image comprising at least one portion of a face of said person (P);
• data (22) from a previously created knowledge database, said previously created knowledge database comprising at least one information related to said person (P) and a plurality of conversation topics, each conversation topic being associated with at least one emotion;
- at least one processor configured to:
i. extract (13) from said at least one signal (S(tₑ)) (21) representative of the emotional status at least one emotional signature (S_{E}(tₑ));
ii. compare (14) said extracted at least one emotional signature (S_{E}(tₑ)) to a previous emotional signature (S_{E}(tₑ-Δt)) of said person (P) extracted at a previous time (tₑ-Δt), said previous emotional signature (S_{E}(tₑ-Δt)) being comprised in said previously created knowledge database;
iii. based on said comparison, obtain (15) a degree of similarity of emotional signatures;
iv. based on said degree of similarity, select (16) at least one conversation topic among said plurality of conversation topics;
v. generate and transmit (17) an engaging message to said person (P) based on said at least one selected conversation topic;
vi. collect (18), from said person (P), at least one current signal (S_{C}(t_{c})) (50) representative of the emotional status generated after reception of said engaging message.

2. The device according to claim 1, wherein said at least processor is further configured to, after collecting said at least one current signal (S_{C}(t_{c})) (50):
vii. extract (19) from said at least one current signal (S_{C}(t_{c})) (50) at least one current emotional signature (S_{CE}(t_{c}));
viii. update (20) said previously created knowledge database using said at least one selected conversation topic and said at least one current emotional signature (S_{CE}(t_{c}));
ix. repeat (21) steps ii. to viii. on said at least one current signal (S_{C}(t_{c})) (50), said at least one current signal (S_{C}(t_{c})) (50) comprising at least one among: a voice signal of said person (P) or an image comprising at least one portion of a face of said person (P);
wherein said at least one output is further configured to provide (22) said updated knowledge database (60).

3. The device according to any one of claims 1 to 2, wherein said selected conversation topic is compared to a conversation topic comprised in the previously created knowledge database to evaluate an emotional status evolution of said person (P).

4. The device according to any one of claims 1 to 3, wherein said knowledge database is updated by adding a couple of information comprising said extracted at least one emotional signature (S_{E}(tₑ)) and said selected at least one conversation topic to said knowledge database.

5. The device according to any one of claims 1 to 4, wherein said extracted emotional signature (S_{E}(tₑ)) is representative of: at least one emotion or a combination of at least two distinct emotions.

6. The device according to any one of claims 1 to 5, wherein said knowledge database is structured according to a predefined ontology taking into account at least one emotional signature and at least one conversation topic.

7. The device according to any one of claims 1 to 6, wherein said knowledge database further comprises textual, multimedia and/or contextual information associated with emotions enabling a representation of said person (P).

8. The device according to any one of claims 1 to 7, wherein selecting said at least one conversation topic is based on a semantic analysis of the information comprised in the knowledge database taking into account said emotional signatures.

9. The device according to any one of claims 1 to 8, wherein the plurality of conversation topics is updated from at least one external data source.

10. The device according to any one of claims 1 to 9, wherein:
- said at least one input is further configured to receive at least one initial signal representative of the emotional status (S(t₀)) of said person (P), said initial signal (S(t₀)) being at least a voice signal of said person (P) or an image comprising at least a portion of the face of said person (P); and
- said at least one processor is further configured to:
- extract from said at least one initial signal (S(t₀)) at least one initial emotional signature (S_{E}(t₀));
- add said initial emotional signature (S_{E}(t₀)) to said previously created knowledge database.

11. The device according to any one of claims 1 to 10, wherein said at least one processor is further configured to:
- periodically select a predefined question from a list of predefined questions;
- generate and transmit an engaging message to said person (P) based on said predefined question;
- collect, from said person (P), at least one current signal (S_{C}(t_{c})) (50) representative of the emotional status generated after reception of said engaging message to said person (P) based on said predefined question, and extract from said at least one current signal (S_{C}(t_{c})) (50) at least one current emotional signature (S_{CE}(t_{c}));
- update said previously created knowledge database using said predefined question and said at least one current emotional signature (Sc_{E}(t_{c})).

12. The device according to any of claims 1 to 11, wherein said at least one signal (S(te)) (21) representative of the emotional status of said person (P) further comprises a motion signal obtained using an inertial unit worn by said person (P).

13. The device according to any of claims 1 to 12, wherein said device 1 is further configured to receive as input at least one result from at least one questionnaire for evaluating a psychiatric status of said person (P), and wherein said at least one result is used to update said knowledge database.

14. The device according to any one of claims 1 to 13, wherein said at least one information related to said person (P) is at least one of the following: age, sex, weight, height, hobby, preference, preferred sport, health data.

15. A computer-implemented method for collecting a data from a person (P) at risk of a psychiatric disorder for a diagnostic, health monitoring and/or a screening purpose(s), said method comprising:
a) receiving (41):
• at least one signal (S(tₑ)) (21) representative of an emotional status of said person (P), said signal (S(tₑ)) (21) being at least a voice signal of said person (P) or an image comprising at least one portion of a face of said person (P);
• data (22) from a previously created knowledge database, said previously created knowledge database comprising at least one information related to said person (P) and a plurality of conversation topics, each conversation topic being associated with at least one emotion;
b) extracting (43) from said at least one signal (S(tₑ)) (21) representative of the emotional status at least one emotional signature (S_{E}(tₑ));
c) comparing (44) said extracted at least one emotional signature (S_{E}(tₑ)) to a previous emotional signature (S_{E}(tₑ-Δt)) of said person (P) extracted at a previous time (tₑ-Δt), said previous emotional signature (S_{E}(tₑ-Δt)) being comprised in said previously created knowledge database;
d) based on said comparison, obtaining (45) a degree of similarity of emotional signatures;
e) based on said degree of similarity, selecting (46) at least one conversation topic among said plurality of conversation topics;
f) generating and transmitting (47) an engaging message to said person (P) based on said at least one selected conversation topic;
g) collecting (48), from said person (P), at least one current signal (S_{C}(t_{c})) (50) representative of the emotional status generated after reception of said engaging message.
